# EUROPEAN PATENT APPLICATION

(11) **EP 1 262 199 A1**
(43) Date of publication of application: **04.12.2002**
(21) Application number: 01201944.4
(22) Date of filing: 23.05.2001
(51) Int. Cl.: A61K 48/00, A61K 121/00, C12N 15/67

(54) **Vectors for enhanced expression of VEGF for disease treatment**

(71) Applicant: Fornix Biosciences N.V., 8243 PM Lelystad (NL)
(72) Inventor: Visser, Tjerk Johannes Anske, 1016 DG Amsterdam (NL); Roossien, Folkert Feiko, 1319 AM Muiden (NL); Ubink-Bontekoe, Carola Jacoba Maria, 8072 DK Nunspeet (NL)
(74) Representative: Prins, Adrianus Willem

(57) **Abstract**

The invention provides a vector which is capable of the expression of a vascular endothelial growth factor wherein said vector comprises a modified pCMV promoter. The invention further provides use of a vector which is capable of and expressing a vascular endothelial growth factor (VEGF) for the regulation of endothelial function, angiogenesis and arteriogenesis. The invention further comprises use of a vector which is capable of the expression of a vascular endothelial growth factor (VEGF) for the prophylactic treatment of arterial diseases and/or bone marrow diseases and/or neural diseases.

SEQUENCE LISTING

<110> Fornix Biosciences N.V.

<120> Vectors for enhanced expression of VEGF for disease
      treatment

<130> P54449EP00

<140> 01201944.4
<141> 2001-05-23

<160> 6

<170> PatentIn Ver. 2.1

<210> 1
<211> 9
<212> DNA
<213> Artificial Sequence

<220>
<223> Description of Artificial Sequence: 9bp nucleic
      acid sequence

<220>
<221> misc_feature
<222> (1)..(9)

<400> 1
acgcgcgct                                                          9

<210> 2
<211> 33
<212> DNA
<213> Artificial Sequence

<220>
<223> Description of Artificial Sequence: PCR-forward
      primer

<220>
<221> misc_feature
<222> (1)..(33)

<400> 2
atcgacatcc agtcaaagcc acgttgtgtc tca                               33

<210> 3
<211> 27
<212> DNA
<213> Artificial Sequence

<220>
<223> Description of Artificial Sequence: PCR reverse
      primer

<220>
<221> misc_feature
<222> (1)..(27)

<400> 3
atagcatgcg agttttcgcc ccgaaga                                      27

<210> 4
<211> 31
<212> DNA
<213> Artificial Sequence

<220>
<223> Description of Artificial Sequence: forward primer

<220>
<221> misc_feature
<222> (1)..(31)

<400> 4
gcggactagt gctgtccctc ttctcttatg a                                31


<210> 5
<211> 31
<212> DNA
<213> Artificial Sequence

<220>
<223> Description of Artificial Sequence: backward
      primer

<220>
<221> misc_feature
<222> (1)..(31)

<400> 5
gccgacgtgc agtctaggtg aagatccttt t                                31


<210> 6
<211> 5874
<212> DNA
<213> Artificial Sequence

<220>
<223> Description of Artificial Sequence: plasmid ph
      VEGF165.MB sequence

<220>
<221> misc_feature
<222> (1)..(5874)

<400> 6
   gaattcgagc tcgccccgtt acataactta cggtaaatgg cccgcctggc tgaccgccca 60
   acgacccccg cccattgacg tcaataatga cgtatgttcc catagtaacg ccaataggga 120
   ctttccattg acgtcaatgg gtggagtatt tacggtaaac tgcccacttg gcagtacatc 180
   aagtgtatca tatgccaagt acgcccccta ttgacgtcaa tgacggtaaa tggcccgcct 240
   ggcattatgc ccagtacatg accttatggg actttcctac ttggcagtac atctacgcgc 300
   gctacgtatt agtcatcgct attaccatgg tgatgcggtt ttggcagtac atcaatgggc 360
   gtggatagcg gtttgactca cggggatttc caagtctcca ccccattgac gtcaatggga 420
   gtttgttttg gcaccaaaat caacgggact ttccaaaatg tcgtaacaac tccgccccat 480
   tgacgcaaat gggcggtagg cgtgtacggt gggaggtcta tataagcaga gctcgtttag 540
   tgaaccgtca gatcgcctgg agacgccatc cacgctgttt tgacctccat agaagacacc 600
   gggaccgatc cagcctccgg gggatcttgg tggcgtgaaa ctcccgcacc tcttcggcca 660
   gcgccttgta gaagcgcgta cgatgaactt tctgctgtct tgggtgcatt ggagccttgc 720
   cttgctgctc tacctccacc atgccaagtg gtcccaggct gcacccatgg cagaaggagg 780
   agggcagaat catcacgaag tggtgaagtt catggatgtc tatcagcgca gctactgcca 840
   tccaatcgag accctggtgg acatcttcca ggagtaccct gatgagatcg agtacatctt 900
   caagccatcc tgtgtgcccc tgatgcgatg cgggggctgc tgcaatgacg agggcctgga 960
   gtgtgtgccc actgaggagt ccaacatcac catgcagatt atgcggatca aacctcacca
   1020
   aggccagcac ataggagaga tgagcttcct acagcacaac aaatgtgaat gcagaccaaa
   1080
   gaaagataga gcaagacaag aaaatccctg tgggccttgc tcagagcgga gaaagcattt
   1140
   gtttgtacaa gatccgcaga cgtgtaaatg ttcctgcaaa aacacagact cgcgttgcaa
   1200
   ggcgaggcag cttgagttaa acgaacgtac ttgcagatgt gacaagccga ggcggtgagg
   1260
   atcctgagaa cttcagggtg agtttgggga cccttgattg ttctttcttt ttcgctattg
   1320
   taaaattcat gttatatgga gggggcaaag ttttcagggt gttgtttaga atgggaagat
   1380
   gtcccttgta tcaccatgga ccctcatgat aattttgttt ctttcacttt ctactctgtt
   1440
   gacaaccatt gtctcctctt attttctttt cattttctgt aactttttcg ttaaacttta
   1500
   gcttgcattt gtaacgaatt tttaaattca cttttgttta tttgtcagat tgtaagtact
   1560
   ttctctaatc actttttttt caaggcaatc agggtatatt atattgtact tcagcacagt
   1620
   tttagagaac aattgttata attaaatgat aaggtagaat atttctgcat ataaattctg
   1680
   gctggcgtgg aaatattctt attggtagaa acaactacac cctggtcatc atcctgcctt
   1740
   tctctttatg gttacaatga tatacactgt ttgagatgag gataaaatac tctgagtcca
   1800
   aaccgggccc ctctgctaac catgttcatg ccttcttctc tttcctacag ctcctgggca
   1860
   acgtgctggt tgttgtgctg tctcatcatt ttggcaaaga attcactcct caggtgcagg
   1920
   ctgcctatca gaaggtggtg gctggtgtgg ccaatgccct ggctcacaaa taccactgag
   1980
   atctttttcc ctctgccaaa aattatgggg acatcatgaa gccccttgag catctgactt
   2040
   ctggctaata aaggaaattt attttcattg caatagtgtg ttggaatttt ttgtgtctct
   2100
   cactcggaag gacatatggg agggcaaatc atttaaaaca tcagaatgag tatttggttt
   2160
   agagtttggc aacatatgcc catatgctgg ctgccatgaa caaaggtggc tataaagagg
   2220
   tcatcagtat atgaaacagc cccctgctgt ccattcctta ttccatagaa aagccttgac
   2280
   ttgaggttag atttttttta tattttgttt tgtgttattt ttttctttaa catccctaaa
   2340
   attttcctta catgttttac tagccagatt tttcctcctc tcctgactac tcccagtcat
   2400
   agctgtccct cttctcttat ggagatccct cgaggagctt tttgcaaaag cctaggtaat
   2460
   gaatcggcca acgcgcgggg agaggcggtt tgcgtattgg gcgctcttcc gcttcctcgc
   2520
   tcactgactc gctgcgctcg gtcgttcggc tgcggcgagc ggtatcagct cactcaaagg
   2580
   cggtaatacg gttatccaca gaatcagggg ataacgcagg aaagaacatg tgagcaaaag
   2640
   gccagcaaaa ggccaggaac cgtaaaaagg ccgcgttgct ggcgtttttc cataggctcc
   2700
   gcccccctga cgagcatcac aaaaatcgac gctcaagtca gaggtggcga aacccgacag
   2760
   gactataaag ataccaggcg tttccccctg gaagctccct cgtgcgctct cctgttccga
   2820
   ccctgccgct taccggatac ctgtccgcct ttctcccttc gggaagcgtg gcgctttctc
   2880
   atagctcacg ctgtaggtat ctcagttcgg tgtaggtcgt tcgctccaag ctgggctgtg
   2940
   tgcacgaacc ccccgttcag cccgaccgct gcgccttatc cggtaactat cgtcttgagt
   3000
   ccaacccggt aagacacgac ttatcgccac tggcagcagc cactggtaac aggattagca
   3060
   gagcgaggta tgtaggcggt gctacagagt tcttgaagtg gtggcctaac tacggctaca
   3120
   ctagaagaac agtatttggt atctgcgctc tgctgaagcc agttaccttc ggaaaaagag
   3180
   ttggtagctc ttgatccggc aaacaaacca ccgctggtag cggtggtttt tttgtttgca
   3240
   agcagcagat tacgcgcaga aaaaaaggat ctcaagaaga tcctttgatc ttttctacgg
   3300
   ggtctgacgc tcagtggaac gaaaactcac gttaagggat tttggtcatg agattatcaa
   3360
   aaaggatctt cacctagact gccagtcaaa gccacgttgt gtctcaaaat ctctgatgtt
   3420
   acattgcaca agataaaaat atatcatcat gaacaataaa actgtctgct tacataaaca
   3480
   gtaatacaag gggtgttatg agccatattc aacgggaaac gtcttgctcg aggccgcgat
   3540
   taaattccaa catggatgct gatttatatg ggtataaatg ggctcgcgat aatgtcgggc
   3600
   aatcaggtgc gacaatctat cgattgtatg ggaagcccga tgcgccagag ttgtttctga
   3660
   aacatggcaa aggtagcgtt gccaatgatg ttacagatga gatggtcaga ctaaactggc
   3720
   tgacggaatt tatgcctctt ccgaccatca agcattttat ccgtactcct gatgatgcat
   3780
   ggttactcac cactgcgatc cccgggaaaa cagcattcca ggtattagaa gaatatcctg
   3840
   attcaggtga aaatattgtt gatgcgctgg cagtgttcct gcgccggttg cattcgattc
   3900
   ctgtttgtaa ttgtcctttt aacagcgatc gcgtatttcg tctcgctcag gcgcaatcac
   3960
   gaatgaataa cggtttggtt gatgcgagtg attttgatga cgagcgtaat ggctggcctg
   4020
   ttgaacaagt ctggaaagaa atgcataagc ttttgccatt ctcaccggat tcagtcgtca
   4080
   ctcatggtga tttctcactt gataacctta tttttgacga ggggaaatta ataggttgta
   4140
   ttgatgttgg acgagtcgga atcgcagacc gataccagga tcttgccatc ctatggaact
   4200
   gcctcggtga gttttctcct tcattacaga aacggctttt tcaaaaatat ggtattgata
   4260
   atcctgatat gaataaattg cagtttcatt tgatgctcga tgagtttttc taatcagaat
   4320
   tggttaattg gttgtaacac tggcagagca ttacgctgac ttgacgggac ggcggctttg
   4380
   ttgaataaat cgaacttttg ctgagttgaa ggatcagatc acgcatcttc ccgacaacgc
   4440
   agaccgttcc gtggcaaagc aaaagttcaa aatcaccaac tggtccacct acaacaaagc
   4500
   tctcatcaac cgtggctccc tcactttctg gctggatgat ggggcgattc aggcctggta
   4560
   tgagtcagca acaccttctt cacgaggcag acctcagcgc cccccccccc cgttcttcgg
   4620
   ggcgaaaact ctcaaggatc ttaccgctgt tgagatccag ttcgatgtaa cccactcgtg
   4680
   cacccaactg atcttcagca tcttttactt tcaccagcgt ttctgggtga gcaaaaacag
   4740
   gaaggcaaaa tgccgcaaaa aagggaataa gggcgacacg gaaatgttga atactcatac
   4800
   tcttcctttt tcaatattat tgaagcattt atcagggtta ttgtctcatg agcggataca
   4860
   tatttgaatg tatttagaaa aataaacaaa taggggttcc gcgcacattt ccccgaaaag
   4920
   tgccacctga cgtctaagaa accattatta tcatgacatt aacctataaa aataggcgta
   4980
   tcacgaggcc ctttcgtctc gcgcgtttcg gtgatgacgg tgaaaacctc tgacacatgc
   5040
   agctcccgga gacggtcaca gcttgtctgt aagcggatgc cgggagcaga caagcccgtc
   5100
   agggcgcgtc agcgggtgtt ggcgggtgtc ggggctggct taactatgcg gcatcagagc
   5160
   agattgtact gagagtgcac catatgcggt gtgaaatacc gcacagatgc gtaaggagaa
   5220
   aataccgcat caggaaattg taagcgttaa tattttgtta aaattcgcgt taaatttttg
   5280
   ttaaatcagc tcatttttta accaataggc cgaaatcggc aaaatccctt ataaatcaaa
   5340
   agaatagacc gagatagggt tgagtgttgt tccagtttgg aacaagagtc cactattaaa
   5400
   gaacgtggac tccaacgtca aagggcgaaa aaccgtctat cagggcgatg gcccactacg
   5460
   tgaaccatca ccctaatcaa gttttttggg gtcgaggtgc cgtaaagcac taaatcggaa
   5520
   ccctaaaggg agcccccgat ttagagcttg acggggaaag ccggcgaacg tggcgagaaa
   5580
   ggaagggaag aaagcgaaag gagcgggcgc tagggcgctg gcaagtgtag cggtcacgct
   5640
   gcgcgtaacc accacacccg ccgcgcttaa tgcgccgcta cagggcgcgt ccattcgcca
   5700
   ttcaggctgc gcaactgttg ggaagggcga tcggtgcggg cctcttcgct attacgccag
   5760
   ctggcgaaag ggggatgtgc tgcaaggcga ttaagttggg taacgccagg gttttcccag
   5820
   tcacgacgtt gtaaaacgac ggccagtgaa ttgtaatacg actcactata gggc
   5874

## Description

The invention relates to the field of medicine. In particular it relates to the treatment of disease more specifically arterial diseases and/or bone marrow diseases and/or neural diseases and/or inflammatory disorders.

In mammalian vascular development mesoderm-derived cells differentiate into endothelial cells that coalesce into blood vessels in a process called vasculogenesis. The formation of new blood vessels from pre-existing endothelium is termed angiogenesis. Several markers are consistently associated with embryonic stem (ES) cell-derived precursor cells having vascular potential, including the vascular endothelial growth factor (VEGF) receptor flk-1, the cell adhesion receptor platelet endothelial cell adhesion molecule (PECAM), and the adhesion molecule VE-cadherin.

VEGF-A functions in the development of embryonic structures during tissue remodelling and for the growth of tumour-induced vasculature. In solid tumors there is a constant requirement for vascular supply. Tumor-associated neovascularization is important for tumor cells to express their critical growth advantage. Experimental and clinical evidence suggests that the process of metastasis, is angiogenesis-dependent. Angiogenesis is a crucial process for tumor growth, metastasis and inflammation. Various angiogenic growth factors and cytokines induce neovascularization in tumors, namely members of the vascular endothelial growth factor (VEGF) and angiopoietin (Ang) gene families. Vascular endothelial growth factor (VEGF) is thought to be the most potent angiogenic factor in numerous malignant tumors and is a prognostic indicator for cancer patients. A strong correlation has been found between VEGF expression and increased tumor microvasculature, malignancy, and metastasis, for example in breast cancer. Vascular endothelial growth factor (VEGF) signaling is required for both differentiation and proliferation of vascular endothelium. VEGF-A stimulates many actions of endothelial cells including proliferation, migration, and nitric oxide release *via* binding to and activation of the two primarily endothelial-specific receptor-tyrosine kinases KDR and Flt-1. KDR and Flt-1 stimulate multiple signal transduction pathways in endothelial cells. These molecules also have in vivo expression patterns that are consistent with their being early markers of vascular lineage. VEGF signaling is critical for blood vessel formation during development.

In mouse VEGF is a 45-kd homodimer produced at sites of vasculogenesis and angiogenesis, and alternative splicing results in 3 different isoforms. The homodimer ofVEGF-165 is the most active form and its properties include mitogenesis, chemotaxis, and permeability for endothelial cells. VEGF binds to 2 high-affinity receptors, flk-1 (VEGFR-2) and flt-1 (VEGFR-1), that are expressed in endothelium. Recently, a third molecule that binds VEGF with high affinity was identified as neuropilin-1, a receptor that also signals in the nervous system through a different ligand. Available data suggest that neuropilin-1 acts as a coreceptor with flk-1 in vascular tissues. Expression patterns of receptors and ligands suggest that VEGF may be a highly specific mediator of blood vessel formation in vivo, and analysis of targeted mutations in the mouse supports this hypothesis. Both flk-1 and fit-1 receptor mutations are recessive embryonic lethals at days 8.5 to 9.5 of gestation. The flk-1 mutation severely impairs vasculogenesis and hematopoiesis, whereas the flt-1 mutation affects vascular organization. Recent findings suggest that VEGF signaling is required for the transition of flk-1+ and PECAM+ cells to vascular endothelial cells that express ICAM-2 and CD34.

Dominantly acting transforming oncogenes are generally considered to contribute to tumor development and progression by their direct effects on tumor cell proliferation and differentiation. The growth of solid tumors beyond 1-2 mm in diameter requires the induction and maintenance of a tumor blood vessel supply, which is attributed in large part to the production of angiogenesis promoting growth factors by tumor cells. The mechanisms which govern the expression of angiogenesis growth factors in tumor cells are largely unknown, but dominantly acting oncogenes are thought to have a much greater impact than hitherto realised. An example of this is the induction of expression of vascular endothelial growth factor/vascular permeability factor (VEGF/VPF) by mutant H- or K-ras oncogenes, as well as v-src and v-raf, in transformed fibroblasts or epithelial cells. Tumor-derived vascular endothelial growth factor (VEGF)/vascular permeability factor (VPF) plays an important role in neovascularization and the development of tumor stroma. Besides VEGF/VPF, mutant ras genes are known to up-regulate the expression of a variety of other growth factors thought to have direct or indirect stimulating effects on angiogenesis, e.g. TGF-beta and TGF-alpha. This effect may be mediated through the ras-raf-MAP kinase signal transduction pathway, resulting in activation of transcription factors such as AP1, which can then bind to relevant sites in the promoter regions of genes encoding angiogenesis growth factors. In principle, similar events could take place after activation or over-expression of many other oncogenes, especially those which can mediate their function through ras-dependent signal transduction pathways.

Excess blood vessel formation contribute to initiating and maintaining many diseases such as chronic inflammatory disorders, tumor growth, restenosis, and atherosclerosis. In contrast insufficient blood vessel formation is responsible for tissue ischemia, as in coronary artery disease. The treatment of vascular disease although greatly improved over recent decades by drug medication, surgical and minimally-invasive techniques, remains limited by vascular proliferative lesions and by our inability to modulate the progression of native disease. An increasing number of patients with advanced coronary artery disease remain symptomatic despite maximal interventional, surgical or medical treatment. Ideally, they would benefit most from additional arterial blood supply to ischemic areas of myocardium. The invention discloses a novel therapeutic strategy for the treatment of vascular disease using the angiogenic growth factor VEGF. Previous therapeutic strategies using VEGF comprising proteins and/or plasmids were not able to produce VEGF polypeptide in a mammalian cells in sufficient quantities to improve neovascularisation or regional mycardial blood flow.

The invention provides a vector which is capable of effecting the expression of a vascular endothelial growth factor (VEGF or a functional equivalent thereof) in the appropriate environment wherein said vector comprises a modified pCMV promoter. The definition 'functional equivalent" (homologue) means that a particular subject sequence varies from the reference sequence by one or more substitutions, deletions, or additions resulting in a sequence that encodes the same activity as VEGF in kind, not necessarily in amount. A vector capable of the effecting the expression of VEGF as used herein is a vector comprising a VEGF nucleic acid, that is at least capable of being expressed in the appropriate environment (i.e. a vector that is at least operative in mammalian systems). Preferably said vector comprises a plasmid. Preferably said modified pCMV promoter comprises the insertion of a 9bp nucleic acid sequence ACGCGCGCT, wherein A is a deoxyadenyl, G is deoxyguanyl, C is deoxycytosyl and T is thymidyl. pCMV promoter refers to the human cytomegalovirus (CMV) promoter. It is understood that (single) base substitutions may be made in said promoter sequence that will not significantly affect the function of the sequence.

The invention also provides a vector capable of effecting expression of VEGF, which lacks CpG-rich regions. Prior art vectors often had ampicillin resistance sequences or other sequences promoting immunogenicity such as CpG-islands. This may have contributed to the lack of efficacy in the prior art.

The invention provides a vector capable of effecting the expression of a vascular endothelial growth factor (VEGF) wherein said vector comprises an antibiotic resistance gene. Preferably said antibiotic resistance gene comprises kanamycin. Even more preferred said vector comprises a 5' HSV translation initiation signal and/or a β-globin splicing/adenylation signal. Also considered are all viral and eukaryotic, preferably mammalian, translation initiation signals and splicing/adenylation signals. Exogenous transcriptional elements and initation codons can be used and also can be of various origins, both natural and synthetic. The invention further provides a vector which is capable of effecting the expression of a vascular endothelial growth factor (VEGF), wherein said vector comprises a ColE1-like replicon and/or an F1 replication origin. ColE1-type replicons (e.g., pBR322) and other ColE1-like replicons (pMB1-, p15A, RSF1030-, and CloDF13-derived) in *E. coli* are considered suitable. F1 replication origin refers to phage F1 origin. All known replication origins previously defined and yet to be defined are deemed suitable. The efficiency of expression may be enhanced by the inclusion of enhancers appropriate for and least operative in mammalian systems.

In a preferred embodiment the invention provides a vector capable of effecting the expression of a vascular endothelial growth factor (VEGF), wherein said vascular endothelial growth factor (VEGF) is derived from a mammal. Preferably said mammal is a human. The invention further provides a vector capable of effecting the expression of a vascular endothelial growth factor (VEGF), wherein said vector comprises a vascular endothelial growth factor (VEGF) nucleic acid. Nucleic acid as used herein refers to an oligonucleotide, nucleotide or polynucleotide, and fragments or portions thereof, and to DNA or RNA of genomic or synthetic origin which may be single- or double-stranded, and represents the sense or antisense strand. It is understood that it is advantageous to increase the production of the endogenous vascular endothelial growth factor (VEGF) in a mammalian system, to promote angiogenesis, for example for the prophylactic treatment of arterial diseases and/or bone marrow diseases and/or neural diseases and/or inflammatory disorders, in particular tissue ischemia especially associated with diabetes. In a preferred embodiment said nucleic acid is operative in mammalian systems.

In the case of mammalian expression vectors, the expression of a nucleic acid of the invention may be driven by a number of previously defined and yet to be defined promoters, including inducible, developmentally regulated and tissue specific promoters. Promoters or enhancers derived from the genomes of mammalian cells are considered suitable. The invention further provides the use of the individual promoter of the nucleic acid of the present invention for this purpose. In particular any promoters of nucleic acids particularly involved in the regulation of endothelial function, angiogenesis or arteriogenesis are deemed suitable.

The invention provides a vector according to the invention wherein said vector can replicate to a high copy number in a bacterial cell. The invention further provides a host cell which comprises a vector according to the invention. The definition host cell as used herein refers to a cell in which an foreign process is executed by bio-interaction, irrespective of the cell belonging to a unicellular, multi-cellular, a differentiated organism or to an artificial cell or cell culture. Preferably said host cell is a mammalian cell, more preferred a human cell. The invention provides a host cell which contains within its genome a recombinant nucleic acid according the invention.

In a preferred embodiment the invention provides use of a vector which is suitable and capable of effecting the expression of a vascular endothelial growth factor (VEGF) according to the invention for the regulation of endothelial function. Vascular endothelial growth factor (VEGF) is also a specific endothelial cell mitogen that stimulates endothelial function and angiogenesis and plays a crucial role in tumor growth/angiogenesis. VEGF has a variety of effects on vascular endothelium, including the ability to promote endothelial cell viability, mitogenesis, chemotaxis, and vascular permeability. Vascular endothelial growth factor-A (VEGF-A) acts on endothelial cells and monocytes, two cell types that participate in the angiogenic and arteriogenic process in vivo. Monocytes are important in arteriogenesis and that their ability to migrate may be critical to the arteriogenic response. VEGF-A stimulates monocyte migration in healthy individuals.

The invention further provides use of a vector which is suitable and capable of effecting the expression of a vascular endothelial growth factor (VEGF) according to the invention for the regulation of angiogenesis. VEGF signaling is critical for blood vessel formation during development. VEGF plays an important role in the development of the vascular system, wound healing, vascularization of tumors, and for angiogenesis in ischemic tissues including the heart. The invention provides for use of a vector according to the invention for promoting neovascularization of ischemic tissues, as a preventative treatment or early stage treatment of cardiovascular diseases. VEGF-A can initiate the process of vascularization by stimulating chemoattraction and proliferation of angioblasts and endothelial cells [i.e. VEGF-A expression can stimulate angiogenic remodeling]. VEGF is also a potent vasodilating agent. The invention provides for use of a vector according to the invention for promoting neovascularization of wounds. VEGF expression is involved not only the formation of a vascular network but also promotes tissue formation. VEGF is also required for the cyclical blood vessel proliferation in the female reproductive tract.

The invention further comprises the use of a vector which is suitable and capable of effecting the expression of a vascular endothelial growth factor (VEGF) according to the invention for the regulation of arteriogenesis. In a preferred embodiment the invention provides the use of a vector according to the invention for the regulation of coronary and peripheral artery angiogenesis. VEGF-A plays a role as an endogenous activator of coronary collateral formation in the human heart. In a preferred embodiment said vector capable of effecting the expression of VEGF according the invention can be used in the prophylactic treatment of myocardial tissue (diseases), and also in the reconstructing process of infarcted myocardial tissue.

The invention further provides use of a vector according to the invention to induce the release of hematopoietic growth factors by bone marrow endothelial cells. VEGF is also required for longitudinal bone growth and endochondral bone formation. VEGF can induce the release of hematopoietic growth factors (GM-CSF) by bone marrow endothelial cells. In a preferred embodiment said vector capable of effecting the expression of VEGF according to the invention can be used to induce the release of hematopoietic growth factors, for example GM-CSF, to accelerate hematologic recovery after bone marrow grafting. Hematopoietic growth factors can mobilize peripheral blood stem cells from the bone marrow to therapeutically intervene in accelerating hematologic recovery. In a preferred embodiment said vector capable of effecting the expression of VEGF according to the invention may be used for the prophylactic treatment of haematological and oncological diseases. Preferably said vector capable of effecting the expression of VEGF according to the invention may be used to release hematopoietic growth factors in tissues after myeloablative chemotherapy and hematopoietic stem cell grafting. This can reduce the mortality related to autologous and allogeneic graft failure (i.e. bone marrow grafting). The invention further provides a vector capable of effecting the expression of VEGF according to the invention may be used to release hematopoietic growth factors which may be used to increase the quality of cytapheresis peripheral stem cell harvesting.

The invention further provides use of a vector capable of effecting the expression of VEGF according to the invention to induce transendothelial progenitor cell migration. Preferably to induce stromal cell-derived factor-1 (SDF-1) driven transendothelial progenitor cell migration. In the presence of VEGF in-vitro stromal cell-derived factor-1 (SDF-1) can drive and thus increase transendothelial progenitor cell migration. This may be due to pore formation (increased endothelial fenestration). Transendothelial migration of progenitors in vitro is substantially enhanced by the chemokine stromal-cell-derived factor-1 (SDF-1), which is produced by bone marrow stromal cells. More primitive progenitors also respond to this chemokine. Transendothelial progenitor cell migration is regulated by adhesion molecules, paracrine cytokines, and chemokines. Mobilizing hematopoietic growth factors stimulate proliferation of hematopoietic cells, which may indirectly result in changes of the local cytokine and chemokine milieu, adhesion molecule expression, and eventually the mobilization of hematopoietic progenitor cells.

The invention provides use of a vector capable of effecting the expression of VEGF according to the invention to increase endothelial fenestration. In tissues outside the brain, vascular endothelial growth factor-A (VEGF) causes vascular hyper-permeability by opening of inter-endothelial junctions and induction of fenestrations and vesiculo-vacuolar organelles (WOs).

The invention provides use of a vector capable of effecting the expression of VEGF according to the invention to modulate neuroblastoma cell growth. VEGF may affect neuroblastoma cell growth directly and could be an autocrine growth factor. In a preferred embodiment said vector capable of effecting the expression of VEGF according the invention can be used in the prophylactic treatment of neural diseases, and in the reconstruction process after neural injury.

### Critical limb ischemia and diabetes mellitus

Critical limb ischemia is one of the most burdensome problems in diabetes treatment, and provides a major challenge for VEGF gene transfer:
- Patients with diabetes mellitus have a 20-fold risk of developing clinical peripheral arterial disease, a 17-fold increased risk of developing foot gangrene, and a 15-fold increased risk of amputation. In the Netherlands in 1992, 1810 amputations were performed. Actually 50% of lower leg amputations in non-trauma patients is performed in diabetes mellitus. Patients with diabetes mellitus have after unilateral amputation a 40-55% risk of amputation of the contralateral limb in the next 5 years. While the mean admission duration for clinical cases with a primary diagnosis of peripheral arterial disease is 12.2 days, this increases to 30 days in diabetes mellitus. Thus, in diabetes mellitus the prevalence of CLI is not only increased, but its course is markedly more serious.
- The localization of macrovascular lesions (stenosis/occlusions) in diabetes mellitus shows a predilection for distal vessels. Especially involvement of vessels below the knee is prominent. On top of this, microvascular involvement is specific for diabetes mellitus. The localization of these lesions makes them less accessible to conventional forms of revascularisation (surgery, PTA, stent).
- The current range of treatment options in CLI in diabetic foot disease is quite small. If possible revascularisation procedures are performed, and in case of superimposed infections aggressive antibiotic treatment is given. However, medical treatment is limited to prostacyclin analogues and attempts to improve tissue oxygenation with rheological means (e.g. Isodex), both with limited success. Initial results from a clinical study show less amputations and better limb survival of as a result of the VEGF treatment for the mentioned disease. Thus gene transfer of a VEGF vector according to the invention is useful for the treatment of tissue ischemia.

The invention further provides use of a vector capable of effecting the expression of VEGF according to the invention in the preparation of a composition. Suitable base for compositions are known in the art. The invention further provides a composition comprising a vector capable of effecting the expression of VEGF according to the invention. Preferably said composition is in a form that can be administered to a mammal. A preferred embodiment is that said composition is suitable for human external application, for example wound healing.

The invention also provides a composition comprising a vector capable of effecting the expression of VEGF according to the invention which is a pharmaceutical. Suitable pharmaceutical compositions are known and they may be in dosage forms such as tablets, pills, powders, suspensions, capsules, suppositories, injection preparations, ointments, eye drops etc.

In a preferred embodiment the invention provides use of a composition according to the invention and/or a vector according to the invention for the treatment of arterial diseases and/or bone marrow diseases and/or neural diseases and/or inflammatory disorders, in particular tissue ischemia especially associated with diabetes. Modes of administration can readily be determined by conventional protocols. A preferable mode of administration is by syringe injection to a localized tissue.

The invention further comprises a method of treatment of arterial diseases and/or bone marrow diseases and/or neural diseases and/or inflammatory disorders and/or wounds comprising administering a vector according to the invention and/or a composition according to the invention with a carrier to a suitable recipient. Preferably said carrier is a pharmaceutically acceptable carrier (e.g. drug carrier system) or inert carrier.

### Examples

Example 1: Construction of a highly efficient VEGF expression plasmid (phBEGF165.MB) without an ampicilline resistant gene.

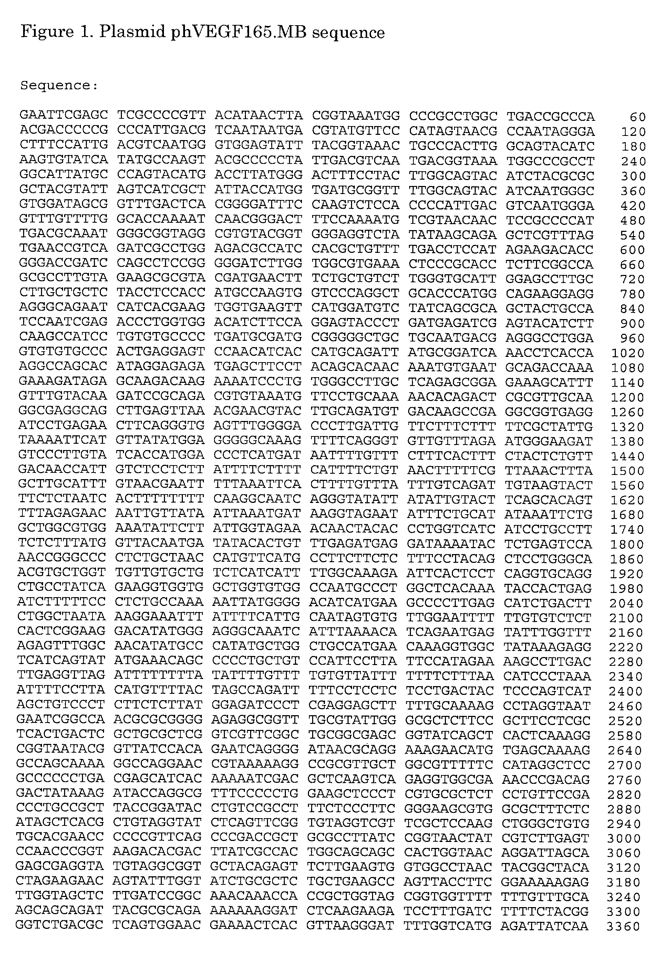

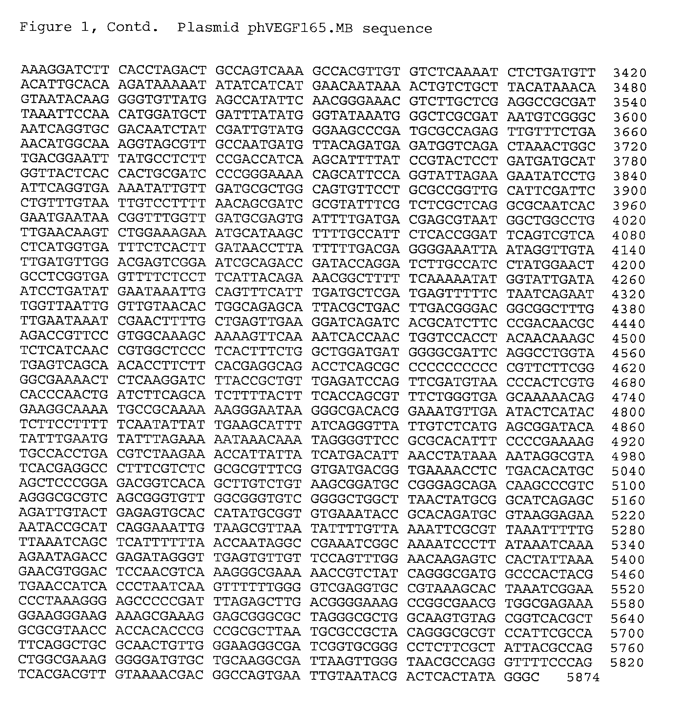

Plasmid phVEGF165.MB (figure 1) was constructed from phVEGF165.SR, which was originally constructed by cloning a VEGF cDNA, obtained by RT-PCR of human vascular smooth muscle cells, into a eukaryotic expression vector (see Severne et al., 1988 and Isner et al., 1996 for details). The plasmid phVEGF165.SR (kindly provided by Isner), was re-sequenced. Besides a few mutations (including in the AhdI site), an almost complete $-lactamase gene was found. The 3' part of the ampicillin resistance gene (751 bp) was deleted out of phVEGF165.SR.

### Deleting of the 3' part of $-lactamase gene (751bp)/Creating AhdI site:

The PCR-forward primer: 5'-ATCGACATCCAGTCAAAGCCACGTTGTGTCTCA-3' (first 2 nucleotides are miscellaneous, followed by an AhdI site), and the PCR reverse primer: 5'-ATAGCATGCGAGTTTTCGCCCCGAAGA-3', were used to amplify kanamycin. For Co1E1-origin the forward primer: 5'-GCGGACTAGTGCTGTCCCTCTTCTCTTATGA-3' and the backward primer: 5'-GCCGACGTGCAGTCTAGGTGAAGATCCTTTT-3' (increased with AhdI) were used.

Both PCR products were ligated to each other, using AhdI, in an pCR-BluntII-TOPO (Invitrogen). And after sequencing, the kanamycin/ori gene was digested with ClaI and BlnI, and ligated into the ClaI/BlnI sites of phVEGF165.SR. This resulted in a plasmid without ampicillin and with a new AhdI restriction site: phVEGF165.MB. The promotor region was changed during growth in the E. Coli DH5-alpha and included in this promoter was a 9bp nucleic acid sequence ACGCGCGCT insertion. Synthesis of VEGF mRNA is driven by the modified cytomegalovirus (CMV) promoter. Downstream of the VEGF coding region is a rabbit β-globin sequence for splicing and polyadenylation. A 5' untranslated thymidine kinase sequence from herpes simplex virus (HSV) is used for translation initiation. The phVEGF165.MB plasmid harbours the kanamycin gene for selection during propagation in *E.Coli*.

| Positions in the plasmid: | | |
|---|---|---|
| 14- 619 | CMV promoter/enhancer | - CMV |
| 620- 682 | 5'HSV translation initiation signal | - 5'HSV |
| 682-1258 | VEGF coding sequence | - VEGF |
| 1259-2180 | (3-globin splicing/polyadenylation signal | - pA |
| 2457-3370 | Co1E1-type replicon | - ori |
| 3388-4623 | kanamycin gene | - kana |
| 4647-4724 | 5' part of $-lactamase gene | |
| 5238-5690 | f1 origin | - f1 ori |
| 5854-5873 | T7 promoter | - T7 |

### Example 2. Detection of the ability of the phVEGF165MB plasmid to transform COS7 cells to produce VEGF protein.

70% confluent COS7 cells were transfected with VEGF plasmid Fugene. After 3 and 4 days the VEGF concentration in the supernatant was detected with a VEGF R&D kit (ELISA). The VEGF production (n=3)on day 3 and 4 of the plasmids without (phVEGF165.RS) and with(phVEGF165.MB)the modified cytomegalovirus (CMV) promoter were respectively 1.3/1.5 and 1.4/1.6 (pg/ml per 10E6 COS cells).

Phase I clinical studies have established that intramuscular gene transfer may be utilized to successfully accomplish therapeutic angiogenesis.

Gene transfer was performed in ten limbs of nine patients with non-healing ischemic ulcers (n=7) and/or rest pain (n=10) due to peripheral arterial disease. A total amount of 4000 µg naked plasmid DNA encoding the secreted 165-amino acid isoform of human VEGF (phVEGF₁₆₅) was injected into ischemic muscles of the affected limb. The average follow-up was 6 ± 3 (range 2 to 11) months. Local intramuscular gene transfer induced no or mild local discomfort up to 72 hours after the injection. Serial CPK measurements remained in the normal range and there were no signs of systemic or local inflammatory reactions. To date, no aggravated deterioration in eyesight due to diabetic retinopathy or growth of latent neoplasm has been observed in any patient treated with phVEGF₁₆₅ gene tranfer. The only complication observed in the trial, was limited to transient lower extremity edema, consistent with VEGF-enhancement of vascular permeability.

*Transgene expression.* Blood levels of VEGF transiently peaked one to three weeks post gene transfer in seven patients amenable for weekly assays. In two patients baseline and/or more than two follow-up blood samples were not achievable. Clinical evidence of VEGF (vascular permeability factor) overexpression was evident by the observation of peripheral edema development (+1 to +4 by gross inspection) in those six patients with ischemic ulcers. In four patients, the edema was limited to the treated limb, while in two patients the contralateral limb was affected as well, albeit less severely. Edema corresponded temporally to the rise in serum VEGF levels.

*Noninvasive arterial testing.* The absolute systolic ankle or toe pressure increased in nine limbs post gene transfer and was unchanged in one limb at the time of the most recent follow-up (p=0.008). The ABI and/or TBI increased from 0.33 ± 0.04 (0.22 to 0.57, p=0.028, [n=10]) at four weeks; to 0.45±0.04 (0.27 to 0.59, p=0.016, [n=10]) at eight weeks; and to 0.48 ± 0.03 (0.27 to 0.67, p=0.017, [n=8]) at 12 weeks. Improvement in the pressure index was sustained, but did not further rise significantly after the second gene transfer. Exercise performance improved in all five patients with rest pain or minor ischemic ulcers, who underwent a graded treadmill exercise. All patients experienced a significant increase in pain-free walking time (2.5 ± 1.1 min pre gene therapy vs 3.8 ± 1.5 min at an average of 13 weeks post gene therapy, p=0.043) and absolute, claudication-limited walking time (4.2 ± 2.1 min vs 6.7 ± 2.9 min, p=0.018). Two patients reached the target endpoint often minutes of exercise.

*Angiography.* Digital subtraction angiography showed newly visible collateral vessels at the knee, calf and ankle levels in six of ten ischemic limbs treated. The luminal diameter of the newly visible vessels ranged from 200 µm to > 800 µm, although most were closer to 200 µm and these frequently appeared as a "blush" of innumerable collaterals. Collaterals did not regress in follow-up angiograms. Magnetic resonance angiography showed qualitative evidence of improved distal flow with enhancement of signal intensity as well as an increase in the number of newly visible collaterals in eight limbs.

*Change in limb status and ischemic rest pain*. Therapeutic benefit was demonstration by regression of rest pain and/or improved limb integrity. The frequency of ischemic rest pain expressed as afflicted nights per week decreased significantly (5.9 ± 2.1 at baseline vs 1.5 ± 2.8 at eight week follow-up, p=0.043), with a slight reduction of analgesic medication (on average from 1.8 to 1.5 analgetics/24h period). Based on criteria proposed by Rutherford, limb status improved in nine of ten extremities treated. Moderate improvement, including *both* an upward shift in the clinical category (at least one clinical category in patients with rest pain and at least two categories to reach the level of claudication in patients with tissue loss) and an increase in the ABI > 0.1 was documented in five cases. In one patient an ischemic ulcer resolved sufficiently to permit placement of a split-thickness skin grafting, leading to absolute limb salvage. In two patients, in whom a major amputation would have been inevitable, retention of a functional foot by a minor (toe) amputation was reached. Minimal improvement, including an upward shift in clinical category or improvement of the ABI > 0.1 was present in another three cases. However, in two patients with and extensive forefoot necrosis and osteomyelitis a below-knee amputation was required despite significant hemodynamic and angiographic improvement. There was one patient with progressive toe gangrene, who remained unchanged from his hemodynamic and angiographic findings. The patient underwent a below-knee amputation eight weeks after gene therapy.

*Immunohistochemistry and molecular analysis*. Tissue specimens derived from one amputee ten weeks after gene therapy showed foci of proliferating endothelial cells. This finding was particularly striking with the fact in mind, that endothelial cell proliferation is nearly absent in normal arteries, is consistent with an estimated endothelias cell turnover time of "thousands of days" is quiescent microvasculature. PCR performed on these samples indicated persistence and widespread distribution of DNA fragments unique to phVEGF₁₆₅. Noteworthy amplification of DNA fragments was shown in muscle and skin samples derived from the site of injection as well as in several muscle samples remote from the site of injection. Southern blot analysis confirmed persistence of intact plasmid DNA in muscle specimen derived from two amputees eight and ten weeks after gene therapy.

### Dose and administration

**1.** Dosage The clinical study is performed as a double blind phase III study in 60 patients. In half of the patients a total dose of 4000 ug ph VEGF₁₆₅ will be given, each patient will receive 4 injections (each with 500 µg phVEGF₁₆₅), each with a volume of 1 ml. This will be repeated after 4 weeks (day 28). The other half of the patients will receive injections with physiologic salt without phVEGF.
**2.** Administration The VEGF gene via a small needle (27 G) is injected directly into the muscle. The spreading of the injectate into the muscle is also monitored in this way. After introduction of the needle 1ml of saline or saline containing 500 µg phVEGF₁₆₅ is injected. This is done at four different injection sites in gastrocnemius and anticus muscles.

## Claims

1. A vector which is capable of effecting the expression of a vascular endothelial growth factor (VEGF or a functional equivalent thereof) in an appropriate environment wherein said vector comprises a modified pCMV promoter.

2. A vector according to claim 2 wherein said modification comprises the insertion of a 9bp nucleic acid sequence ACGCGCGCT, wherein A is a deoxyadenyl, G is deoxyguanyl, C is deoxycytosyl and T is thymidyl.

3. A vector according to claim 1 or 2 wherein said vector comprises an antibiotic resistance gene.

4. A vector according to claim 3 wherein said antibiotic resistance gene comprises kanamycin.

5. A vector according to anyone of claims 1-4 wherein said vector comprises a 5' HSV translation initiation signal and/or a (β-globin splicing/adenylation signal.

6. A vector according to anyone of claims 1-5 wherein said vector comprises a ColE1-like replicon and/or an F1 replication origin.

7. A vector according to anyone of claims 1-6 wherein said vascular endothelial growth factor (VEGF) is derived from a mammal.

8. A vector according to claim 7 wherein said mammal is a human.

9. A vector according to anyone of claims 1-8 wherein said vector comprises a vascular endothelial growth factor (VEGF) nucleic acid.

10. A vector according to anyone of claims 1-9 wherein said nucleic acid is operative in mammalian systems.

11. A vector according to claim 10 wherein said vector can replicate to a high copy number in a bacterial cell.

12. A host cell which comprises a vector according to anyone of claims 1-11.

13. A host cell according to claim 12 which is human.

14. Use of a vector according to anyone of claims 1-11 in the preparation of a medicament for the regulation of endothelial function.

15. Use of a vector according to anyone of claims 1-11 in the preparation of a medicament for the regulation of angiogenesis.

16. Use of a vector according to anyone of claims 1-11 in the preparation of a medicament for the regulation of arteriogenesis.

17. Use of a vector according to anyone of claims 1-11 in the preparation of a medicament to induce the release of hematopoietic growth factors by bone marrow endothelial cells.

18. Use of a vector according to anyone of claims 1-11 in the preparation of a medicament to induce transendothelial progenitor cell migration.

19. Use of a vector according to anyone of claims 1-11 in the preparation of a medicament to increase endothelial fenestration.

20. A composition comprising a vector according to anyone of claims 1-11.

21. A composition according to claim 20 which is a pharmaceutical composition.

22. Use of a composition according to claim 20 or 21 and/or a vector according to anyone of claim 1-11 for the treatment of arterial diseases and/or bone marrow diseases and/or neural diseases and/or inflammatory disorders, in particular tissue ischemia especially associated with diabetes.

23. A method of treatment of arterial diseases and/or bone marrow diseases and/or neural diseases and/or inflammatory disorders, comprising administering a vector according to anyone of claims 1-11 and/or a composition according to claim 20 or 21 with a carrier to a suitable recipient.
